# EUROPEAN PATENT SPECIFICATION

(11) **EP 0 337 390 B1**
(45) Date of publication and mention of the grant of the patent: **17.02.1993**
(21) Application number: 89106410.7
(22) Date of filing: 11.04.1989
(51) Int. Cl.: A61F 2/16

(54) **Intraocular optical system**
Intraokulares optisches System
Système d'optique intraoculaire

(30) Priority: 11.04.1988 CS 2470/88
(43) Date of publication of application: 18.10.1989
(73) Proprietor: CESKOSLOVENSKA AKADEMIE VED, Praha 1 (CS)
(72) Inventor: Sulc, Jiri, Dipl.-Ing., Praha 4 (CS); Krcová, Zuzana, Dipl.-Ing., Praha 7 (CS)
(74) Representative: Beetz & Partner Patentanwälte

(56) References cited:
- EP-A- 0 162 573
- EP-A- 0 212 616
- GB-A- 2 199 672
- US-A- 4 409 691
- US-A- 4 710 194
- US-A- 4 790 847
- US-A- 4 842 601

## Description

The invention pertains to an intraocular optical lens system for insertion into the capsula lentis after removal of the natural eye lens as specified in the preamble of Claim 1. Such a system is known from US-A-4 409 691. This document discloses an intraocular lens that provides accommodation in response to contraction and relaxation of the ciliary body. Accommodation is achieved by motion of the lens, or of an element of a lens system, alternatively toward and away from the fovea. Means are provided for biasing the lens, or the movable element, toward the fovea so that when the ciliary body is fully relaxed the lens is at its closest position to the fovea, and as the ciliary body contracts it counters the bias and causes the lens to move away from the fovea and toward the cornea.

Intraocular lenses, above all hard lenses made from poly(methyl methacrylate), which are inserted into the capsula lentis after removal of the natural lens, usually as a consequence of cataract, reached a considerable perfection but still have some shortcomings. Some of them, for example, the tendency to the growth of cells on the surface of the lenses and irritation of living tissues, predominantly in the places of the contact of supporting projections (haptics), and the tendeny to form strong light reflexes, could be overcome by a surface hydrophilization by forming a soft surface layer having a swelling gradient, but the shortcoming of the necessity to make a long incision at operation still remains unsolved.

A considerable improvement could be achieved by soft intraocular lenses based on hydrophilic gels which can be inserted in a suitably deformed state into the preserved capsula lentis through a small incision, where the lens then assumes its final, correct shape and required position.

A disadvantage of soft intraocular lenses based on hydrophilic gels used so far in comparison with the original natural lens consists in the difficult accommodation to various distances, especially in elderly patients. This is due to the fact that hydrogels need a larger power for deformation than can be developed by the pertinent eye muscles. On the other hand, very soft lenses have only a low refractive index of light and low strength, and a large volume, which may develop the so-called secondary glaucoma due to clogging of the natural passages.

It is the object of the present invention to provide an intraocular optical lens system for insertion into the eye after removal of the natural lens which is not subject to the shortcomings of prior art intraocular lenses, particularly irritation of adjacent tissues, can be introduced into the eye through a small incision, fits into the original capsula lentis and allows accommodation.

The above object is achieved according to the claims. The dependent claims relate to preferred embodiments.

The intraocular optical system according to the invention for insertion into the capsula lentis after removal of the natural eye lens is defined in Claim 1, namely as
(A) a hollow body having an outer shape following the inner shape of the capsula lentis at least in the main lines and leaning against the inner wall of the capsula lentis and keeping it in a moderately tensioned state,
   and
(B) comprising
   - a front element,
   - a rear element,
   - an elastically deformable element provided between the front element and the rear element,
      and
   - one to four lenses arranged such as to be placed in the main axis of the eye, at least one of the lenses being provided such as to move axially at contraction and release of the accommodation muscles of the eye and thus to change its position between the retina and the cornea.

The elastically deformable element has preferably elasticity in the direction of the optical or geometrical axis of the system.

The intraocular optical system according to the present invention is preferably made partially or in its entirety from a homopolymer or a copolymer on the basis of acrylates, methacrylates and/or hydroxyalkyl acrylates and/or hydroxyalkyl methacrylates, such as hydroxyethyl methacrylate (HEMA).

At least one of the lenses may be placed in the optical system concerned outside the geometric center of the system and of the capsula lentis.

The intraocular optical system of the present invention is preferably made partially or in its entirety from a biocompatible material, for example, from silicon elastomers, hydrogels, and the like, which are advantageously elastic and have a shape memory, for example, partially dried hydrogels, and which may be hydrophilized at the surface.

The intraocular optical system can be inserted into the eye through a small incision, i.e. smaller than 3 mm, in a deformed state, which can be realized when it is made from a material having a glass-transition temperature T_{g} in the range of -5 to 45 °C, or from a material the T_{g} value of which can be adjusted before implantation in such a way that it meets the above condition, for example, by swelling a hydrophilic gel into a non-equilibrium state.

In this case, the system is heated above its glass-transition temperature, deformed into a rod-like shape with a diameter smaller than 3 mm, and cooled below this temperature while retaining its rod-like shape. After insertion into the eye, the system spreads into its original shape due to the body temperature or rehydration. The lenses of the optical system can be inserted into the eye in the same way.

The intraocular optical system according to the invention allows to use lenses from an arbitrary material, i.e. not only from a hydrogel but also from hard acrylic polymers such as poly(methyl methacrylate), without the known shortcomings, particularly irritation of adjacent tissues, becoming operative. On the contrary, the application of hard lenses with a high refractive index enables to reduce their dimension in such a way that the posterior chamber of the eye remains unfilled, and the whole system fits into the original capsula lentis from which the natural, turbid lens was removed, for example, by phaco-emulsification.

It is of advantage, if at least one of the lenses of the optical system is made of a synthetic polymer having a refractive index of light of at least 1.336.

The intraocular optical system may have the shape of ellipsoid of revolution provided on its circumference with an equatorial slot with a row of holes, and may be provided on its front side with an opening which has a smaller diameter than that of the opening in the capsula lentis.

The front part and the rear part of the system may be realized in the form of rings with a diameter < 9 mm or as parts of hollow bodies of revolution, such as a sphere, a paraboloid, an ellipsoid, and the like. The lenses (optical elements) may be integrated therewith, whereby the lenses and their case may form one single piece. The elastically deformable element may have the shape of fibers, strips, a spiral, or a corrugated body.

One single lens may be provided either in the rear or in the front element; if two lenses are used, the case be provided both in the front and rear elements. In the latter case, both lenses move away from one another at accomodation for near sight, and approach one another at accommodation for long-distance sight. In addition thereto, the system may be complemented by provision of one or more further intraocular lenses.

It is important that the accommodation proceeds only by shifting the substitute lens in the eye axis forward and backwards, similarly as in photographic cameras, and not by changing the shape of the lens as it is in a healthy eye, where the shift of the lens in the eye axis is smaller and occurs in parallel with the change in its curvature, i.e. with the change in its optical power. The so-called zoom effect, which takes place in a healthy eye during accomodation to a smaller extent, may be also attained with the system according to this invention with two or more lenses, without parallel change in the optical power of the individual lenses.

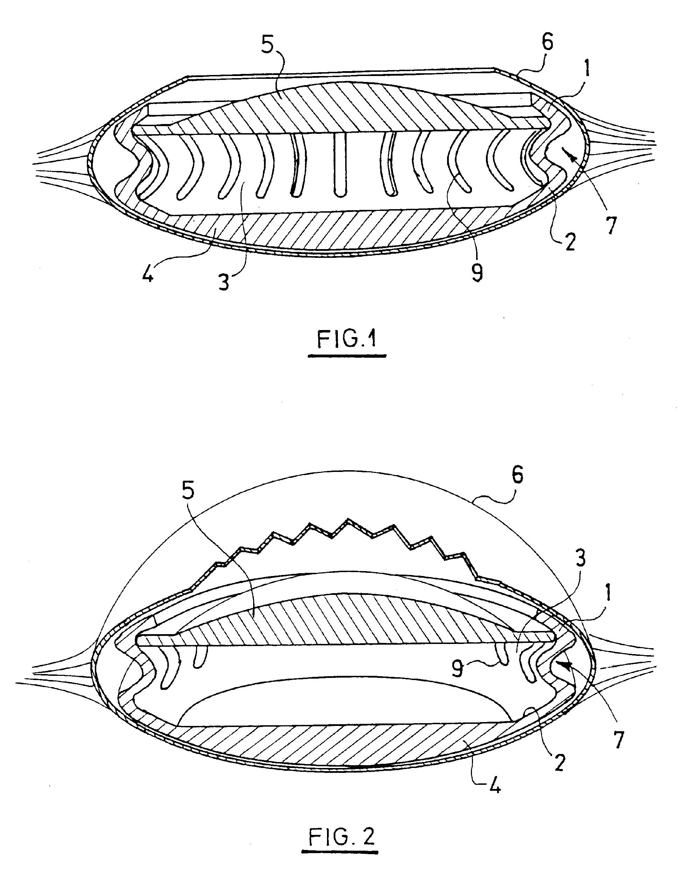

In the following, the intraocular optical system according to the invention will be explained with reference to specific embodiments shown in Figures 1 to 6.

Figure 1 shows a section of a first embodiment of the intraocular optical system 7 according to the present invention which consists of a front element 1, a ring-shaped elastically deformable element 3 comprising an equatorial slot provided with holes 9 at the circumference, and a rear element 2 comprising the lens 4. The lens 5 is inserted into the front element 1 of the system 7 the front opening of the front element 1 being somewhat smaller than the diameter of the front lens 5. The whole system 7 is placed in the capsula lentis 6.

Figure 2 shows the same section as in Figure 1, viewed from above across.

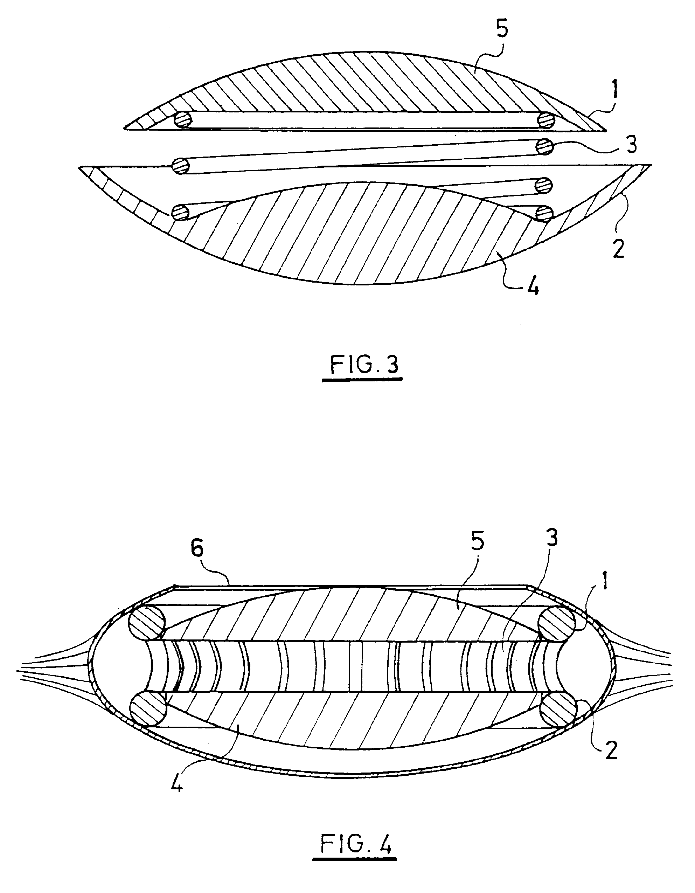

A sectional view of another embodiment of the intraocular optical system of this invention is shown in Figure 3, in which the front element 1 comprising the lens 5 and the rear element 2 with integrated lens 4 are connected by means of the elastically deformable element 3 realized as a spring which simultaneously centers the lenses 4, 5 or the front and rear elements 1, 2, respectively.

Figure 4 represents a section through an intraocular optical system 7 of the invention, where the front element 1 and the rear element 2 are shaped as rings connected to the elastically deformable element 3 also forming a ring. The lenses 4 and 5 are inserted into radially inner slots. The whole system is placed in the capsula lentis 6.

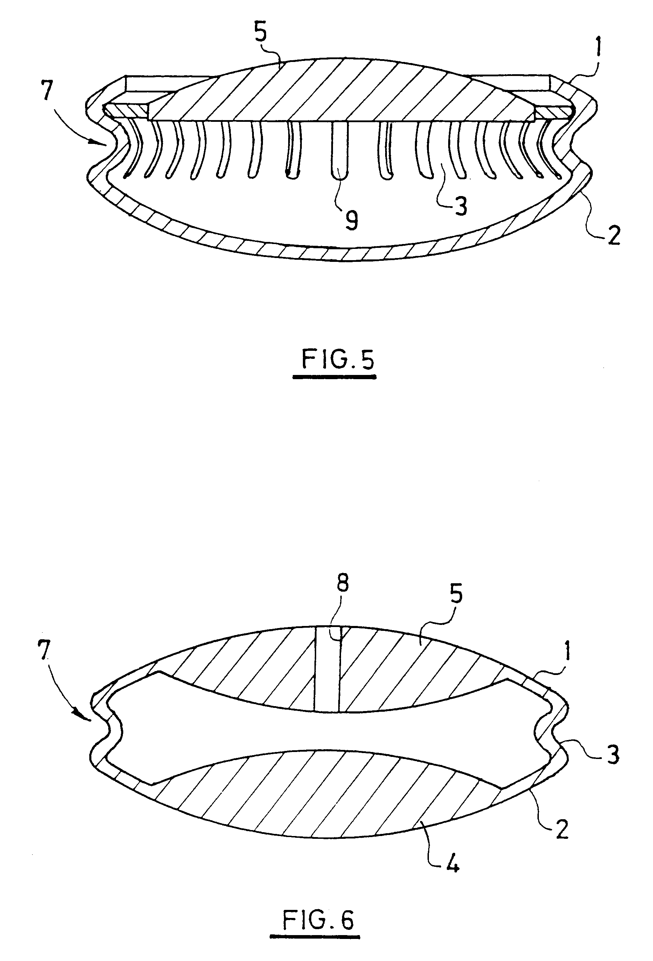

The intraocular optical system 7 shown in Figure 5 in a sectional view consists of the front element 1 and the rear element 2 having the shape of a part of an ellipsoid of revolution and the elastically deformable element 3 provided with an equatorial slot comprising a row of holes 9. The lens 5 is inserted into the front element 1 in a manner similar to that of Figure 1.

Figure 6 shows a section of another embodiment of the intraocular system 7 where the lenses 4, 5, the front element 1, the rear element 2 and the elastically deformable element 3 are monolithically integrated and form one single piece. The hole 8 in the central part of the front lens 5 allows the chamber liquor to circulate.

As may be seen from Figure 6, the front and/or back lens or front and/or rear element may be provided with a central opening, and/or the elastically deformable element may comprise an equatorial slot with a row of holes for liquid passage. The elastically deformable element 3 may be or may not be complemented with a centrically provided spiral spring or other elastic connection elements, where liquids can flow in between. The lenses 4, 5 may be fixed in the rear element 2 or in the front element 1 of the system 7 or in both parts, or may be held in the eye axis by radial or oblique fibers. The oblique fibers are longer than the straight radial fibers and may be therefore easily elastically stretched.

In the most simple arrangement, the system 7 may comprise two ring-shaped elements 1, 2 connected with the elastically deformable element 3, the space therebetween enabling the flow of surrounding liquid. These rings preferably have a diameter of less than 9 mm.

The elastically deformable element 3 may have the form of fibers, strips, a spiral or a corrugated body so that the capsule lentis is moderately and uniformly tensioned by these rings. The elements 1, 2 may also be parts of hollow bodies of revolution, such as a sphere, a paraboloid, an ellipsoid, and the like. They may be made from various biocompatible materials, advantageously of elastic material with shape memory, for example, partially dried hydrogels which may be hydrophilized at the surface, for example, by partial hydrolysis or sulfonation, which may be completed by a partial surface esterification with multifunctional alcohols containing, even after cross-linking esterification, additional free hydrophilic groups, silicone composites, and the like.

It is advantageous to provide an opening in the front element 1 or the front lens 5 of the system 7 which is larger than the hole in the front part of the capsula lentis formed at the extraction of the natural lens. The non-uniform edges of the hole are not mechanically stressed in this way.

The intraocular optical system 7 and/or the lenses 4, 5 may contain a drug in their material, which is released after implantation. Such drugs may be cytostatics or antibodies liquidating cells on the inner wall of the capsula lentis which cause a secondary cataract by producing the lens materials, corticoids, antibiotics, and the like. These drugs disappear after a certain time.

The front lenses 5 of the system 7 may be provided in the center with a hole of a diameter of 0.05 to 1 mm which enables a better communication between the anterior and posterior chambers of the eye and prevents from the formation of a secondary glaucoma. This hole does not affect the optical quality of the image.

The intraocular optical system 7 according to the invention may be manufactured in various ways similar to the manufacture of intraocular lenses, viz. by turning, rotation casting, or dipping.

It is advantageous to produce the system 7 in a way similar to that of the manufacture of intraocular lenses according to GB-A-2 199 672, so that its glass-transition temperature T_{g} is from -5 to 45 °C. A system on that basis may be deformed at a temperature above T_{g} to a form suitable for implantation and cooled down to fix this deformed shape. After insertion into the capsula lentis 9, the system 7 relaxes by post-swelling and warming up to the temperature of the eye and thus acquires the desired final shape.

## Claims

1. Intraocular optical system (7) for insertion into the capsula lentis after removal of the natural eye lens, comprising at least one lens (4,5) arranged such as to be placed in the main axis of the eye and provided such as to move axially at contraction and release of the accommodation muscles of the eye and thus to change its position between the retina and the cornea, and
an elastically deformable element (3) to be deformably actuated by the contraction of the accommodation muscles of the eye,
**characterized** in that said intraocular system
(A) is a hollow body having an outer shape following the inner shape of the capsula lentis (6) at least in the main lines for leaning against the inner wall of the capsula lentis (6) and keeping it in a moderately tensioned state,
and
(B) comprises
- a front element (1),
- a rear element (2), wherein said
elastically deformable element (3) is provided between the front element (1) and the rear element (2),
and
- one to four lenses (4, 5) in total arranged such as to be placed in the main axis of the eye, at least one of the lenses (4, 5) being provided such as to move axially at contraction and release of the accommodation muscles of the eye and thus to change its position between the retina and the cornea.

2. The intraocular optical system (7) according to claim 1, characterized in that at least one of the lenses (4, 5) is fixed in the axis of the system by the elastically deformable element (3) and/or the front element (1) and/or the rear element (2).

3. The intraocular optical system (7) according to claim 1 or 2, characterized in that the front element (1) and/or the rear element (2) and/or the elastically deformable element (3) are rings or parts of hollow bodies of revolution such as a sphere, a paraboloid, an ellipsoid, and the like.

4. The intraocular optical system (7) according to one of claims 1 to 3, characterized in that one or more of the lenses (4, 5) are connected to or integrated with the front element (1) or the rear element (2) to form one single piece.

5. The intraocular optical system (7) according to one of claims 1 to 4, characterized in that the front element (1) and/or the rear element (2) are connected to or integrated with the elastically deformable element (3).

6. The intraocular optical system (7) according to one of claims 1 to 5, characterized in that the elastically deformable element (3) is provided with openings (9) allowing the flow of liquid.

7. The intraocular optical system (7) according to one of claims 1 to 6, characterized in that the elastically deformable element (3) is provided with a circumferential slot.

8. The intraocular optical system according to one of claims 1 to 7, characterized in that the front element (1) or the front lens (5) and/or the back lens (4) are provided with an opening (8) having a diameter which is smaller than that of the hole in the capsula lentis (6).

9. The intraocular optical system (7) according to one of claims 1 to 8, characterized in that the elastically deformable element (3) has elasticity in directions parallel to the optical or geometrical axis of the system.

10. The intraocular optical system (7) according to one of claims 1 to 9, characterized in that the elastically deformable element (3) has the form of fibers, strips, a spiral or of a corrugated body.

11. The intraocular optical system (7) according to one of claims 1 to 10, characterized in that at least one of the lenses (4, 5) is placed outside the geometrical center of the system and of the capsula lentis (6).

12. The intraocular optical system (7) according to one of claims 1 to 11, characterized in that it consists partially or in its entirety of one or more biocompatible materials, particularly of silicon elastomers, hydrogels, preferably elastic hydrogels having shape memory, partially dried hydrogels hydrophilized at the surface, a material having a glass transition temperature (T_{g}) of -5 to 45 °C and/or a material the glass transition temperature of which is adjusted in the state before insertion into the eye to a required value, e.g. by swelling a hydrogel into a non-equilibrium state.

13. The intraocular optical system (7) according to claim 12, characterized in that it is present before insertion into the eye in a deformed state of rod-like shape having a diameter < 3 mm.

14. The intraocular optical system (7) according to one of claims 1 to 13, characterized in that at least one of the lenses (4, 5) is made from a synthetic polymer having a refractive index ≧ 1.336.

15. The intraocular optical system (7) according to one of claims 1 to 14, characterized in that it is made partially or in its entirety from a homopolymer or a copolymer on the basis of acrylates, methacrylates and/or hydroxyethyl methacrylate.

16. The intraocular optical system (7) according to one of claims 1 to 15, characterized in that one or all of the lenses (4, 5) are provided with a center hole having a diameter of 0.05 to 1 mm.

17. The intraocular optical system (7) according to one of claims 1 to 16, characterized in that it contains drugs which are capable of being released into the eye after implantation.

## Patentansprüche

1. Intraokulares optisches System (7) zum Einsetzen in die Capsula Lentis nach dem Entfernen der natürlichen Augenlinse, mit wenigstens einer Linse (4, 5), die auf der Hauptachse des Auges angeordnet und bei Zusammenziehung und Entspannung der Akkomodationsmuskeln des Auges axial bewegbar ist und somit ihre Position zwischen der Retina und der Cornea ändert, und mit einem elastisch verformbaren Element (3), das durch die Zusammenziehung der Akkomodationsmuskel des Auges verformbar betätigt wird,
dadurch **gekennzeichnet**,
daß das intraokulare System
(A) ein Hohlkörper ist, dessen äußere Form der inneren Form der Capsula Lentis (6) wenigstens in den Hauptlinien folgt und sich an die Innenwand der Capsula Lentis (6) anschmiegt und diese in mäßig gespanntem Zustand hält, und
(B) der folgendes aufweist:
- ein vorderes Element (1),
- ein hinteres Element (2), wobei das elastisch verformbare Element (3) zwichen dem vorderen Element (1) und dem hinteren Element (2) angeordnet ist, und
- ein bis vier Linsen (4, 5), die in ihrer Gesamtanordnnung auf der Hauptachse des Auges angeordnet sind, wobei wenigstens eine der Linsen (4, 5) bei Zusammenziehung und Entspannung der Akkomodationsmuskel des Auges axial bewegbar ist, und somit ihre Position zwischen der Retina und der Cornea verändert.

2. Intraokulares optisches System (7) nach Anspruch 1,
dadurch gekennzeichnet,
daß wenigstens eine der Linsen (4, 5) durch das elastisch verformbare Element (3) und/oder das vordere Element (1) und/oder das hintere Element (2) auf der Achse des Systems festgelegt ist.

3. Intraokulares optisches System (7) nach Anspruch 1 oder 2,
dadurch gekennzeichnet,
daß das vordere Element (1) und/oder das hintere Element (2) und/oder das elastisch verformbare Element (3) gebildet ist durch Ringe oder Teile von hohlen Rotationskörpern, wie eine Kugel, ein Paraboloid, ein Ellipsoid und dergleichen.

4. Intraokulares optisches System (7) nach einem der Ansprüche 1 bis 3,
dadurch gekennzeichnet,
daß eine oder mehrere der Linsen (4, 5) zur Bildung eines einzigen Teils mit dem vorderen Element (1) oder dem hinteren Element (2) verbunden oder hiermit einstückig ausgebildet sind.

5. Intraokulares optisches System (7) nach einem der Ansprüche 1 bis 4,
dadurch gekennzeichnet,
daß das vordere Element (1) und/oder das hintere Element (2) mit dem elastisch verformbaren Element (3) verbunden oder hiermit einstückig ausgebildet ist.

6. Intraokulares optisches System (7) nach einem der Ansprüche 1 bis 5,
dadurch gekennzeichnet,
daß das elastisch verformbare Element (3) Öffnungen (9) für den Durchtritt eines Flüssigkeitsstroms aufweist.

7. Intraokulares optisches System (7) nach einem der Ansprüche 1 bis 6,
dadurch gekennzeichnet,
daß das elastisch verformbare Element (3) mit einem Umfangsschlitz versehen ist.

8. Intraokulares optisches System (7) nach einem der Ansprüche 1 bis 7,
dadurch gekennzeichnet,
daß das vordere Element (1) oder die vordere Linse (5) und/oder die hintere Linse (4) mit einer Öffnung (8) versehen ist, deren Durchmesser kleiner als derjenige des Lochs in der Capsula Lentis (6) ist.

9. Intraokulares optisches System (7) nach einem der Ansprüche 1 bis 8,
dadurch gekennzeichnet,
daß das elastisch verformbare Element (3) parallel zur optischen oder geometrischen Achse des Systems elastisch ist.

10. Intraokulares optisches System (7) nach einem der Ansprüche 1 bis 9,
dadurch gekennzeichnet,
daß das elastisch verformbare Element (3) die Form von Fasern, Streifen, einer Spirale oder eines gewellten Körpers hat.

11. Intraokulares optisches System (7) nach einem der Ansprüche 1 bis 10,
dadurch gekennzeichnet, daß wenigstens eine der Linsen (4, 5) außerhalb der geometrischen Mitte des Systems und der Capsula Lentis (6) angeordnet ist.

12. Intraokulares optisches System (7) nach einem der Ansprüche 1 bis 11,
dadurch gekennzeichnet,
daß es teilweise oder insgesamt besteht aus einem oder mehreren biokompatiblen Materialien, insbesondere aus Siliconelastomeren, Hydrogelen, vorzugsweise elastischen Hydrogelen mit Formerinnerungsvermögen, aus teilweise an der Oberfläche hydrophilen getrockneten Hydrogelen, aus einem Material mit einer Glasübergangstemperatur (T_{g}) von -5 bis 45 °C und/oder aus einem Material, dessen Glasübergangstemperatur im Zustand vor dem Einsetzen in das Auge auf einen gewünschten Wert eingestellt wird, zum Beispiel durch Quellen eines Hydrogels in einen Ungleichgewichtszustand.

13. Intraokulares optisches System (7) nach Anspruch 12,
dadurch gekennzeichnet,
daß es vor dem Einsetzen in das Auge in einem verformten stangenförmigen Zustand mit einem Durchmesser von < 3 mm vorliegt.

14. Intraokulares optisches System (7) nach einem der Ansprüche 1 bis 13,
dadurch gekennzeichnet,
daß wenigstens eine der Linsen (4, 5) aus einem synthetischen Polymeren mit einem Brechungsindex von ≧ 1,336 hergestllt ist.

15. Intraokulares optisches System (7) nach einem der Ansprüche 1 bis 14,
dadurch gekennzeichnet,
daß es teilweise oder insgesamt hergestellt ist aus einem Homopolymeren oder einem Copolymeren auf der Basis von Acrylaten, Methacrylaten und/oder Hydroxyethylmethacrylat.

16. Intraokulares optisches System (7) nach einem der Ansprüche 1 bis 15,
dadurch gekennzeichnet,
daß eine oder alle Linsen (4, 5) mit einem zentralen Loch mit einem Durchmesser von 0,05 bis 1 mm versehen ist bzw. sind.

17. Intraokulares optisches System (7) nach einem der Ansprüche 1 bis 16,
dadurch gekennzeichnet,
daß es Arzneimittel enthält, die nach dem Implantieren in das Auge freigesetzt werden können.

## Revendications

1. Système optique intraoculaire (7) à insérer dans la capsule de la lentille après enlèvement de la lentille naturelle (cristallin) de l'oeil, comportant au moins une lentille (4, 5) disposée pour être placée dans l'axe principal de l'oeil et prévue pour se déplacer axialement avec la contraction et le relâchement des muscles d'accommodation de l'oeil et ainsi modifier sa position entre la rétine et la cornée, et un élément déformable élastiquement (3) se déformant sous l'action de la contraction des muscles d'accommodation de l'oeil, caractérisé en ce que le système intraoculaire
A. est un corps creux ayant une forme extérieure suivant la forme intérieure de la capsule de la lentille (6) au moins généralement, pour se reposer contre la paroi de la capsule de la lentille (6) et la maintenir dans un état de tension modéré, et
B. comprend
- un élément frontal (1),
- un élément arrière (2) dans lequel l'élément déformable élastiquement (3) est prévu entre l'élément frontal (1) et l'élément arrière (2),
et
- une des quatre lentilles (4, 5) au total est disposée pour être dans l'axe principal de l'oeil, au moins une des lentilles (4, 5) étant prévue pour se déplacer axialement lors de la contraction et du relâchement des muscles d'accommodation de l'oeil et ainsi pour changer de position entre la rétine et la cornée.

2. Système optique intraoculaire (7) selon la revendication 1, caractérisé en ce qu'au moins une des lentilles (4, 5) est fixée dans l'axe du système par l'élément déformable élastiquement (3) et/ou l'élément frontal (1) et/ou l'élément arrière (2).

3. Système optique intraoculaire (7) selon la revendication 1 ou 2 caractérisé en ce que l'élément frontal (1) et/ou l'élément arrière (2) et/ou l'élément déformable élastiquement (3) sont des anneaux ou des parties de corps creux de révolution tels que sphère, paraboloïde, ellipsoïde, et analogue.

4. Système optique intraoculaire (7) selon l'une des revendications 1 à 3 caractérisé en ce qu'une ou plus des lentilles (4, 5) sont connectées ou intégrées avec l'élément frontal (1) ou l'élément arrière (2) pour constituer une pièce unique.

5. Système optique intraoculaire (7) selon l'une des revendications 1 à 4 caractérisé en ce que l'élément frontal (1) et/ou l'élément arrière (2) sont connectés ou intégrés avec l'élément déformable élastiquement (3).

6. Système optique intraoculaire (7) selon l'une des revendications 1 à 5 caractérisé en ce que l'élément déformable élastiquement (3) est prévu avec des ouvertures (9) permettant l'écoulement de liquide.

7. Système optique intraoculaire (7) selon l'une des revendications 1 à 6 caractérisé en ce que l'élément déformable élastiquement (3) est prévu avec une fente circonférentielle.

8. Système optique intraoculaire selon l'une des revendications 1 à 7 caractérisé en ce que l'élément frontal (1) ou la lentille frontale (5) et/ou la lentille arrière (4) sont prévus avec une ouverture (8) ayant un diamètre inférieur au diamètre du trou de la capsule de la lentille (6).

9. Système optique intraoculaire (7) selon l'une des revendications 1 à 8 caractérisé en ce que l'élément déformable élastiquement (3) a son élasticité dans une direction parallèle à l'axe optique ou géométrique du système .

10. Système optique intraoculaire (7) selon l'une des revendications 1 à 9 caractérisé en ce que l'élément déformable élastiquement (3) a la forme de fibre, de ruban, d'une spirale ou d'un corps strié.

11. Système optique intraoculaire (7) selon l'une des revendications 1 à 10 caractérisé en ce qu'au moins une des lentilles (4, 5) est placée à l'extérieur du centre géométrique du système et de la capsule de lentille (6).

12. Système optique intraoculaire (7) selon l'une des revendications 1 à 11 caractérisé en ce qu'il est constitué partiellement ou dans son intégralité d'un ou plusieurs matériaux biocompatibles, particulièrement d'élastomère de silicone, d'hydrogel, préférentiellement d'hydrogel élastique ayant une mémoire de forme, d'hydrogel partiellement déshydraté hydrophilisé en surface, un matériau ayant une température de solidification T_{g} de - 5 à 45° C et/ou un matériau dont la température de solidification est ajustée en l'état avant l'insertion dans l'oeil à une valeur requise, par exemple en gonflant un hydrogel dans état de non équilibre.

13. Système optique intraoculaire (7) selon la revendication 12 caractérisé en ce qu'il se présente, avant insertion dans l'oeil, dans un état déformé ayant la forme d'un barreau d'un diamètre < 3 mm.

14. Système optique intraoculaire (7) selon l'une des revendications 1 à 13 caractérisé en ce qu'au moins une des lentilles (4, 5) est constituée d'un polymère synthétique ayant un indice de réfraction ≧ 1,336.

15. Système optique intraoculaire (7) selon l'une des revendications 1 à 14 caractérisé en ce qu'il est constitué partiellement ou dans son intégralité d'un homopolymère ou d'un copolymère à base d'acrylate, méthacrylate et/ou hydroxyéthyl méthacrylate.

16. Système optique intraoculaire (7) selon l'une des revendications 1 à 15 caractérisé en ce que l'une ou toutes les lentilles (4, 5) sont prévues avec une ouverture centrale ayant un diamètre de 0,05 à 1mm.

17. Système optique intraoculaire (7) selon l'une des revendications 1 à 16 caractérisé en ce qu'il contient des médicaments qui sont capables d'être libérés dans l'oeil après implantation.
